Europäisches Patentamt

(19) European Patent Office

Office européen des brevets

(11) Publication number: **0 008 826**
A2

# (12) EUROPEAN PATENT APPLICATION

(21) Application number: **79200462.4**

(22) Date of filing: **20.08.79**

(51) Int. Cl.³: **G 01 N 15/00**
**C 12 M 1/34, C 12 Q 1/04**
**C 12 Q 1/24**

(30) Priority: **18.08.78 DK 3667/78**

(43) Date of publication of application:
**19.03.80 Bulletin 80/6**

(84) Designated Contracting States:
**AT BE CH DE FR GB IT LU NL SE**

(71) Applicant: **A/S N. Foss Electric**
**Slangerupgade 69**
**DK-3400 Hillerod(DK)**

(72) Inventor: **Spinell, Max**
**Hellebakkevej 6**
**Gadevang DK-3400 Hillerod(DK)**

(72) Inventor: **Riisgaard, Steen**
**Kovangen 340**
**DK-3480 Fredensborg(DK)**

(74) Representative: **Smith, Sydney**
**Elkington and Fife High Holborn House 52/54 High**
**Holborn**
**London WC1V 6SH(GB)**

(54) **Method and apparatus for counting bacteria in a bacteria-containing suspension, method for fluorescence staining of bacteria, and method for separating bacteria from a sample.**

(57) A suspension of fluorescence-stained bacteria is applied as a film, preferably of a thickness of 2 - 7 $\mu$m, on the edge of a rotating disc, and the film is illuminated with U.V. light. Through a microscope, light impulses from bacteria in the film passing a zone of preferably 2 - 4 $\mu$m transverse to direction of movement of the disc edge are transferred to a transducer, and the electrical signals from the transducer are discriminated according to size and signals exceeding a predetermined size are counted. Disc preferably has a diameter of 8 - 12 cm and rotates at 10 - 40 rpm. Fluorochrome dye is preferably acridine orange or ethidium bromide. Method for staining comprises increasing contrast between bacteria and background by incubating with a proteolytic enzyme, or incubating in a nutrient medium containing an antibiotic preventing division of bacteria, or adding formaldehyde when staining with acridine orange. Separation of bacteria from a sample comprises centrifugation with a higher density liquid system comprising continuous or discontinuos density gradient, especially in a dish-like centrifuge in which sample passes over two-component liquid system to move bacteria to interface between components.

./...

EP 0 008 826 A2

Fig. 1.

Method and apparatus for counting bacteria in a bacteria-containing suspension, method for fluorescence staining of bacteria, and method for separating bacteria from a sample.

The present invention concerns a method and an apparatus for counting bacteria in a sample, a method for fluorescence staining of a bacteria suspension with the purpose of counting the bacteria, and a method for separating bacteria from a sample which, in addition to bacteria, contains other suspended particles and/or emulsified particles.

Counting of bacteria in various materials, for example foodstuffs, has previously been performed by the so-called "plate count" method, in which a representative part of a sample, optionally subsequent to dilution, is spread on a culture substrate, and the number of colonies is assessed visually after incubation. This method is time-consuming, as already the incubation takes several days. The present invention provides a method by which the counting of bacteria in a large number of samples may be performed very quickly and with high accuracy and reproducibility.

The method of the invention for counting bacteria in a bacteria-containing suspension comprises
staining said bacteria with a fluorochrome dye,
rotating a circular-cylindrical surface part about its axis,
applying a film of suspension containing the stained bacteria on the said rotating surface part,
exposing said film to fluorescence-activating radiation,
detecting light signals emitted from said stained bacteria,

transforming said light signals to electrical signals, selecting said electrical signals exceeding a predetermined size, and counting said selected signals.

The above-mentioned method which can be considered an advanced "dynamic microscopy" method may be performed in an apparatus which is described in great detail below and which comprises

a rotatable suspension-receiving member having a circular-cylindrical surface part, typically a rotating disc, the edge of which constitutes the circular-cylindrical surface part,

suspension application means for applying a film of the suspension on the cylindrical surface part, typically a measuring syringe having a suitable nozzle pointing towards the edge of the rotating disc,

radiation means for irradiating the suspension film so as to activate fluorescence from the stained bacteria, typically a UV light source,

transducer means for converting light signals into electrical signals, typically a photomultiplier or a diode or diode array,

counting means for counting electrical signals,

means for passing light signals emitted by the fluorescence-stained bacteria to the transducer means, and discriminator means for discriminating the electrical signals produced by said transducer means according to size so as to only pass signals exceeding a predetermined value to the counting means.

In the following description of the invention, the rotatable suspension-receiving member having a circular-cylindrical surface part is described, for brevity, as the evident practical embodiment thereof constituted by a rotating disc, the edge of which constitutes the surface part in question.

The principle of the dynamic microscopy counting method of the present invention comprises applying a well defined amount per time unit of the suspension in the form of a film on the edge of the disc which rotates at a constant rotational speed and counting the light pulses emitted from the bacteria passing a narrow stationary zone extending transversely to the direction of rotation of the disc edge.

One condition for a successful counting of bacteria with a reliable result utilizing the method of the invention is that the bacteria are so well stained that the light emitted from the bacteria is considerably stronger than the light from the background, in other words, stronger than the light from the liquid phase of the suspension with possible solid impurities, and that the light emitted from the bacteria is transduced as accurately as possible into electrical signals. In the practical embodiments of the method discussed herein, the light emitted from the bacteria is transferred to a transducer by means of a microscope, and it has been found to be very important to the accurate transduction that the light is transferred relatively well-focused, in other words as a relatively sharp picture, to the transducer. According to the invention, it has been found that a satisfactory focusing is obtainable when the suspension film laid out on the disc edge has an average thickness of 2 - 7 µm during the counting operation, preferably an average thickness of 4 - 6 µm. In this connection, the average thickness is to be understood as the average thickness of the suspension film, taken across the cross-section of the film. The suspension film will be higher at the middle and will have a decreasing height towards its two parallel edges. It cannot be precluded that by certain especially beneficial combinations of the power of the available radiation source and the fluorochrome dye used, a somewhat greater average thickness of the suspension can be accepted, but the range of 2 - 7 µm mentioned above has been found to be critical with the combinations used so far. Usually, the film laid out on the disc rim has a width of about 400 - 800 µm, and the average thickness of the film can suitably be assessed through calculation on the basis of this width, the circumference and the rotational speed of the disc, and the volume amount of the suspension which is applied on the disc edge per time unit.

In accordance with the above-mentioned critical importance of the thickness of the suspension film in order to obtain a good focusing, it is also to be noted that the disc proper must necessarily establish a stable support for the film in relation to the focusing of the microscope, that is, the radius variation along the circumference of the disc must be extremely small, according to the invention at the most 2 µm, preferably at the most 1 µm.

It is evident that the reliability of the counting result is also dependent on maximum uniformity in the application of the suspension on the disc edge. Various factors are of importance in this connection. Firstly, the disc must be made of a material which allows a suitable and uniform adhesion of the suspension film, and it has found in this connection that hard glass or hard metal are suitable materials. The application of the suspension on the disc edge must be performed through a nozzle having a size which is adapted to the desired dimensions for the suspension film, and it has been found that application through a nozzle, the orifice of which has a diameter of about 200 µm, is suitable. In order to retain the suspension film in its path on the disc edge, it is suitable that the disc is kept at a temperature which promotes weak evaporation from the suspension; it is presumed that this evaporation gives rise to microscopic "rims" or "walls" of solid material in each edge of the suspension film, and that these edges contribute to retain the suspension film. When the suspension in an aqueous liquid, such as will most often be the case, it is suitable that the disc is kept at a temperature of about 40 - 50°C.

The diameter and rotational speed of the rotating disc must be so chosen that the suspension can be applied in a suitable way and remains in place with stationary dimensions, in other words, the suspension should neither run off because of too slow rotation or be slung off because of too fast rotation, and it has been found that a diameter of 8 - 12 cm for the rotating disc and a rotational speed of 10 - 60, preferably 10 - 40, rpm are suitable parameters. Within these intervals for rotational speed, the highest rotational speeds will, of course, be able to result in the fastest measurement of a sample, but these highest rotational speeds will also incur strict requirements with respect to the strength of the light emitted from the bacteria, and a suitable compromise seems to be a rotating disc with a diameter of about 100 mm at a rotational speed of about 15 rpm.

When a new suspension sample is to be applied on the disc edge, it would take unreasonably long time to obtain the necessary liquid contact between the disc rim and the orifice through which the suspension is applied if the suspension is initially applied at the rate corresponding to the desired dimensions of the suspension film during the counting, and for this reason, the application of each suspension sample is, according to the invention, preferably performed in two phases, a higher amount of suspension being dosed per time unit at the beginning of the application of the suspension in order to obtain fast contact with the disc edge, whereafter, prior to the counting, the dosage is reduced to correspond to the film thickness desired during the counting operation. It has been found suitable to keep an initial dosage corresponding to a film thickness of 15 - 20 μ. In order to obtain a reliable counting, the rotating disc and the device, typically a measuring syringe, with which the suspension is applied on the disc edge, are driven by electrical motors which are operated synchronously with each other to ensure uniform application of the suspension film on the disc edge. A higher initial dosage of the suspension together with the desired synchronization with the motor driving the disc may be obtained by connecting the measuring syringe with an electric motor which rotates at a constant rate corresponding to the film thickness desired during the counting, and with an additional electric motor rotating at a higher rate which corresponds to the initial dosage, the last mentioned motor being only engaged during the initial dosage. Another interesting embodiment uses one step motor which may be operated at two levels of speed.

According to the invention, it is suitable that the bacteria suspension is applied on the disc edge through a nozzle having an orifice diameter of about 200 μm and the suspension should be freed for any particles having such a size that these would be able to disturb the application through the nozzle. For this reason, it is preferred that the suspension, prior to the application, is passed through a filter having a pore size of about 10 - 20 μm.

It is of decisive importance to the reliability of the measurement that there will be no transfer errors, that is, errors caused by mixing up of the suspension sample to be measured with the previous sample or with flushing liquids. To avoid such transfer errors, it must of course be seen that each suspension sample passes through the apparatus in sufficiently large amount to secure that the volume of the sample on which the measurement is performed can really be considered as representative and uncontaminated with previous samples or flushing liquids. It has been found that a suitable volume of the suspension sample to be applied for the counting proper is about 2 - 10 μliter, for example about 5 μliter, and that the total sample size, including the pre-run, is about 6 ml. Subsequent to the application of each suspension sample on the disc and counting thereof, it is preferred not only to perform a thorough flushing of the measuring syringe, the filter and the lines through which the suspension has passed, but also to perform a ventilation, so that the passageways mentioned are substantially free from liquids when the new suspension sample is introduced. Such a ventilation is of special importance to the passageways in the apparatus which cannot be made of polytetrafluoroethylene. Polytetrafluoroethylene is known to permit an efficient flushing away of a liquid sample contained for example in a polytetrafluoroethylene tube by means of the liquid subsequently introduced, as there is no tendency to retaining a laminar "shell" of the previous liquid on the walls of the tube, but these properties are not possessed by for example silicone rubber, while on the other hand silicone rubber is a suitably elastic material for use in for example lines in the apparatus which constitute part of valves.

It has been found that the method of the invention has a broad "dynamic range", that is, a very broad possible range within which the concentration of bacteria in the suspension may be without any deterioration in the counting result due to noise in the electronic components of the apparatus or incorrect counting caused by emission from the background, etc. Excellent results have been obtained at concentrations of the bacteria suspension over 4 potencies of 10, that is from $10^4$ - $10^8$ bacteria/ml. Of course, it is preferred that the concentration of bacteria in the suspension to be measured is in this range which in itself is very broad. If it should be found that a sample has a higher concentration of bacteria than $10^8$ bacteria/ml, it should, therefore, suitably be diluted to a concentration in this range.

Depending on the purpose of the counting performed, it may be of interest to count the total number of bacteria in the sample, including viable and dead bacteria, or it may be of interest to perform a counting to obtain maximum conformity with "viable plate count". The last manufacturer adjustment of the apparatus of the invention is usually an adaption of the discriminator level to the desired type of counting. Thus, when it is desired to obtain maximum conformity with "viable plate count", the counting result obtained by the process of the invention is compared with a counting result obtained through the classical "viable plate count" method, and the discriminator level method is adjusted until maximum conformity is obtained. In case there should still be a minor systematic deviation in certain concentration ranges or at certain types of suspensions, this can be adjusted for through an algorithm which may suitably be microprocessor means incorporated in the apparatus.

The staining of the bacteria in the suspension in which the bacteria are to be counted is suitably performed with a dye resulting in a strong emitted light and having as low a tendency as possible to stain the background, including possible protein-containing or other particles in the suspension which are not bacteria. Fluorochrome dyes which have been found suitable in this connection are, for example, acridine orange which gives a strong emitted light, and ethidium bromide, which results in a less strong emitted light, but which, on the other hand, has less tendency to stain the background.

The staining with acridine orange or ethidium bromide may, in principle, be performed in a manner known per se, for example by mixing, into the bacteria-containing suspension, a solution of acridine orange or ethidium bromide (0.001 per cent) in a physiological medium at a pH of 7.5 in the ratio 1:1, but one aspect of the present invention is a method for fluorescence staining of a bacteria-containing suspension with the purpose of counting the bacteria, in particular with the purpose of counting the bacteria by the method described above, and this aspect of the invention is characterized by performing a modification treatment of the suspension to increase the intensity of the fluorescence staining of the bacteria in relation to the background and/or reduce the number of stainable particles which are not bacteria. Such a modification treatment may be performed in various manners.

One modification treatment of this invention which contributes very considerably to eliminate the possibility of including, in the counting, signals derived from protein-containing impurities in the suspension which are not bacteria, and often to increase the intensity of the staining of the bacteria comprises modification of the suspension by incubation with a proteolytic enzyme prior to staining the suspension with the fluorescence dye. According to the invention, the proteolytic enzyme used is suitably papain, trypsin, a neutrase or, preferably, subtilisin or a subtilisin-like enzyme, and a suitable range of the concentration of the enzyme is $0.6 \times 10^{-3}$ - $60 \times 10^{-3}$ Anson units/ml. When a subtilisin is used, this may suitably be subtilisin produced by Bacillus licheniformis which has been found to be useful with good results in a concentration of about $3 \times 10^{-3}$ - $6 \times 10^{-3}$ Anson units/ml at a pH of about 7.5 - 9.5, for example 7.5 - 9 or preferably 8.5 - 9 at a temperature of 40 - 55°C, preferably 45 - 50°C. When papain is used as the proteolytic enzyme, the same concentration as mentioned above may be used at a pH of about 5 - 8 and at a temperature of about 30 40°C. Papain has been found to be suitable for modification treatment of urine. When a neutrase is used as the proteolytic enzyme, the concentration is usually the same as stated above, the pH is suitably 6 - 8, and the temperature range is suitably 30 - 50°C.

The buffer used in the enzyme treatment is suitably a borate, carbonate or Tris buffer. The incubation with enzyme is performed for a period of time which is suitable for obtaining the desired effect, for example a period from about 10 minutes to half an hour. After the incubation, the modified suspension is stained. The modification treatment is of particular interest in connection with staining with acridine orange as acridine orange has a pronounced tendency to stain the background and non-bacteria particles. When the fluorescence dye used is ethidium bromide, which has less tendency to stain background etc., the modification treatment may not be so critical.

One modification treatment which has been found particular useful when the staining is performed with acridine orange, irrespective of whether or not the suspension to be stained has been incubated with an enzyme such as described above, is the addition of formaldehyde simultaneously or substantially simultaneously with the staining with acridine orange. The addition of formaldehyde results in a marked increase of the intensity of the staining. When staining with acridine orange using formaldehyde, one suitably works at a temperature of 15 - 30°C, and it is suitable to use a concentration of formalin of about 1.5 per cent and a concentration of acridine orange of about $3 \times 10^{-4}$ per cent. Often the simultaneous addition of acridine orange and formaldehyde results in a doubling of the intensity of the light emitted from the bacteria.

A special modification treatment and staining comprises adding, to the suspension, about 0.02 N oxalic acid at pH 4 and thereafter heating to about 100°C and subsequently adding EDTA, formaldehyde and acridine orange in a buffer at a pH of about 9. This also results in an increase of the light emitted from the bacteria, but this method is not preferred as it may have a tendency to also result in a rather strong staining of background impurities.

A special modification treatment according to the invention comprises incubating the bacteria, prior to the fluorescence staining, in a nutrient medium containing such antibiotic under such conditions that the bacteria to be stained will grow, but will not divide to any substantial extent. Through this, the bacteria to be stained will simply become larger, and there will, also with respect to size, be a larger difference between dead and viable bacteria. In addition, it appears that this treatment, like the enzyme treatment, makes the bacterial wall more susceptible to the staining with the fluorescence dye. Such incubation may suitably be performed during a period of about 1/2 hour at about 30°C, and it is important that the nutrient medium provides such stabilized osmotic environment that the bacteria will not be ruptured during the incubation. A stabilized osmotic environment conforming with these conditions may be established by addition of sugar to the nutrient medium, for example sucrose in a concentration of about 10 per cent, but it may also be obtained through a salt content in the nutrient medium, for example 1.5 per cent NaCl. Various antibiotics are known which under the conditions stated have the effect that the bacteria will grow, but will not divide, for example chloramphenicol and penicillins such as ampicillin, benzyl penicillin, cloxacillin, dicloxacillin or combinations thereof.

It may be necessary to use a combination of antibiotics to ensure that all bacteria which may occur in the sample will grow, but will not divide, in other words, that all bacteria which may occur in the sample will subject to substantially the same change with respect to selective stainability in relation to the background.

The nutrient medium used during the incubation with the antibiotic is suitably a rich nutrient medium for culturing bacteria, for example a NIH-broth from Difco or N-broth from Oxoid, but it may be suitable that the nutrient medium, apart from its glucose component, is diluted, for example to about 1/10 of it prescribed concentration. The incubation in the presence of an antibiotic may be combined with the above-mentioned incubation with a proteolytic enzyme, in other words the substrate in which the incubation of the bacteria is performed contains both a proteolytic enzyme of the above kind and in the above concentration intervals, and the antibiotic. Irrespective of whether the antibiotic is used per se altogether with the enzyme, it should be used in such a concentration that the desired effect is obtained, and for ampicillin and benzyl penicillin, for example, this concentration is 0.1 - 0.01 mg/ml.

The substrate in which the enzyme and/or antibiotic treatment is performed is suitably prepared fresh every day by dissolution of the constituents of the substrate, as the liquid substrate in its form ready for use will not be storage stable for any longer time. One aspect of the present invention is a composition which may be used in an easy and simple way for the daily preparation of the substrate in question, this composition comprising, in solid form, one or more antibiotics in combination with rich nutrient substrate components and optionally a buffer salt. This composition may be in the form of a tablet or a powder which is preferably weighed out in unit dosages. The composition suitably contains an additional component which provides a stabilized osmotic environment as mentioned above, for example sucrose, and hence, a composition of this kind may suitably contain sucrose in a concentration corresponding to 10 per cent in the dissolved, ready-for-use form of the composition. Also other sugars may be used for establishing the stabilized osmotic environment. Another possibility is that the composition contains sodium chloride in a concentration corresponding to 1.5 per cent sodium chloride in the dissolved, ready-for-use form of the composition.

The bacteria suspension, the bacteria content of which is to be determined by the method of the invention, may be of various origins, but is typically a sample from a food article or a sample for medical investigation purposes such as a sample of a body fluid or urine. If the sample is, for example, urine, it will usually be immediately suitable for fluorescence staining in the manner described above, but it will often be desirable to perform a modification treatment thereof through incubation with an enzyme to remove the influence from any protein-containing particles in the urine which are not bacteria. If the material, the bacteria content of which is to be determined, is a nutrient article, for example milk or a milk product or meat or a meat product, it is necessary to perform a separation treatment to obtain, from the product in question, a bacteria suspension which is suitable for being subjected to the method of the invention, in other words a suspension of bacteria which contains as little other stainable particles as possible.

Hence, one aspect of the present invention comprises a method for separating bacteria from a liquid sample which, in addition to the bacteria, contains other suspended particles and/or emulsified particles, especially a foodstuff suspension such as a meat product suspension or milk, especially with the purpose of preparing a

suspension for counting the bacteria therein by the above-described counting method, and this separation method comprises applying the sample on a layer of a liquid having a density which is higher than the density of the sample, centrifuging the system constituted by the said layer and the sample applied thereon so as to move bacteria from the sample into a portion of the liquid layer, and thereafter separating the bacteria-containing portion of the said liquid layer. As mentioned above, the sample subjected to this separation treatment must be a liquid sample, and if the product, the bacteria content of which is to be determined, is a solid product, for example a meat product, it must therefore first be subjected to a homogenization treatment in a manner known per se in which it is converted into a suspension.

The above-mentioned separation method may be performed using the high density liquid placed as a column in a centrifuge tube. To obtain the best separation of the bacteria-containing gradient from the liquid phase column after the centrifugation, it is suitable that the liquid phase contains a density gradient which may be discontinous or continuous. The density gradient may, for example, be a concentration gradient containing a methyl cellulose, a dextran or a Ficoll. In accordance with a special embodiment of the method, the liquid phase column comprises an upper phase having low viscosity and a lower phase having higher viscosity. In this way, the bacteria can relatively easy penetrate into the liquid phase column, that is, through the upper phase having low viscosity, while the bacteria are retained in a relatively well-defined place in the lower phase having higher viscosity. Suitable substances which show on the one hand high density and on the other hand low viscousity are certain radioopaque substances such as sodium diatrizoate, and an especially suitable liquid phase column may, starting from the top thereof, comprise an about 4.5 per cent solution of sodium diatrizoate, an about 7 per cent solution of dextran 500, an about 12 per cent solution of dextran 500 and an about 15 per cent solution of dextran 500.

When the sample to be applied on the liquid phase column is milk, it has been found suitable, for elimination of casein lumps, to mix the milk with a buffer (for example a phosphate buffer or a tris buffer) containing ethylene diaminetetraacetic acid (EDTA) and it has also been found suitable that the mixture on the liquid phase column has a temperature of about 40 - 50°C.

The centrifugation of the system comprising the sample applied on the liquid phase column is suitably performed at about 300 - 5000 g. When the sample is milk, the gradient containing the bacteria can be suctioned off as the complete gradient column lying immediately over the fraction containing the main part of the somatic cells of the milk when the centrifugation has been performed. In practice, the liquid column may have a height of for example 3 - 4 mm up to several cm.

In accordance with a particularly interesting embodiment of this separation method, the liquid layer comprises at least two liquid components having different densities, the density of the lower layer being higher than the density of the bacteria and the density of the upper layer being lower than the density of the bacteria so that the bacteria are moved to a portion of said liquid layer at the interface between said liquid components. To obtain this, it is necessary that other cells in the suspension which might sediment at the interface together with the bacteria are removed. For example, in milk or urine samples, white blood cells and other somatic cells would collect at the interface together with the bacteria. Such cells, however, but may be selectively lysed by diluting in distilled water, optionally containing a detergent which will selectively attack eucariotic cells, such as digitonin. This lysing treatment results in such small fractions of the somatic cells that these fractions will hardly sediment together with the bacteria in the centrifugation, and to the extent they do, will hardly tend to interfere with the counting as the signals they emit are too small to be passed by the discriminator.

A particularly interesting separation method according to the invention comprises centrifuging the system constituted by sample and liquid layer in a dish-like centrifuge container having an upper opening defined by a radially inwardly extending rim portion having a minimum radial width substantially equal to the radial thickness of said liquid layer, said sample being supplied to the bottom portion of said centrifuge container and the centrifuged sample being discharged through said upper opening. In this manner, a larger amount of sample can be passed continuously or intermittently over the liquid layer, and the bacteria contained in the sample will be moved therefrom into the interface between a high density component and a low density component in the liquid layer to obtain separation and concentration of the bacteria from the sample. One particular advantage of this embodiment of the separation method of the invention is that a small centrifuge may be used for separating and concentrating the bacteria from relatively large volumes of sample, and after the passage of the desired amount of sample over the liquid layer, the total liquid layer, which now contains the bacteria, can be removed quantitatively from the centrifuge, the deposition of the bacteria at the interface securing that the bacteria will not adhere to the centrifuge. In a preferred embodiment of this special technique, the sample is supplied to the centrifuge container through a supply tube extending through the upper opening of the centrifuge container to a position adjacent to the bottom thereof, which means that there is no need for designing complex slide couplings for any supply channels rotating with the centrifuge. The liquid is introduced from the supply tube at the middle of the centrifuge container and will then be accelerated up as it passes from the middle portion of. the centrifuge to the edge portion thereof. At the moment any sample part has been accelerated up to the rotational velocity at which the centrifuge with the liquid layer rotates, the sample part will be discharged from the upper part of the centrifuge. In this way, the sample will pass in thin layer moving upwardly over the liquid layer. The supply of the sample to the centrifuge container should take place at a substantially uniform rate to secure that to part of the sample is discharged before it has been allowed to deliver its content of bacteria to the liquid layer. For example, in a centrifuge container of a diameter of 8 cm rotating at 25000 rpm, a 20 ml sample can be effectively treated in as little as 30 seconds.

According to a particular aspect of the invention, it is possible to obtain separation of bacteria from a sample and simultaneous staining of the bacteria, without any dying of the liquid in which the bacteria are present, by performing separation of the bacteria from a sample as described above with utilization of a particular dye, this dye being a dye which is formed in situ from two components, one component being introduced in the sample, and the other component being introduced in the liquid phase column. As dye for this purpose one may use, for example, the dyes formed by reaction of europium salts with the ligand 4,4,4-trifluoro-1-(2-thienyl)-1,3-butandione, which is commonly termed thenoyltriflouroacetone or TTA. The resulting chelate is suitably termed europium TTA $(Eu(TTA)_3)$. In this case a europium salt, for example europium nitrate, acetate, or chloride, may be introduced in the sample, and TTA may be introduced in the liquid phase column. It is suitable that the europium salt is incorporated in the sample in a concentration of about $10^{-3}$ molar, and that TTA is dissolved in the liquid phase column in a concentration of about $10^{-2}$ molar. When this combination is used for the staining the excitation filter in the apparatus according to the invention should be a filter which permits the passage of light having a wavelength below 400 nm, and the emission filter should be a filter permitting the passage of light from a wavelength of 595 nm.

If the bacteria in a bacteria suspension which is to be counted, especially in the method described above, adhere to each other, this may give rise to error in the counting results, and one aspect of the present invention comprises a method of avoiding this by dispersing such suspension through subjecting it to a homogenization treatment. According to the invention, it has been found that an efficient homogenization treatment may be performed by passing the suspension through an orifice of a small diameter, for example a cannula having a diameter of at the most 1 mm, suitably a cannula having a diameter of 0.5 - 0.8 mm, the rate of the passage being for example ½ - 2 ml per second.

In the drawings,
Fig. 1 is a front view of an apparatus of the invention for counting bacteria,
Fig. 2 is a diagram illustrating the separation of bacteria from a bacteria-containing sample and the staining of the bacteria,
Fig. 3 is a diagram of the apparatus shown in Fig. 1,
Fig. 4 illustrates, in large magnification, part of the film applied on the disc edge and the observation of this film, and

Fig. 5 illustrates an embodiment of a centrifuge for use in a preferred separation method of the invention.

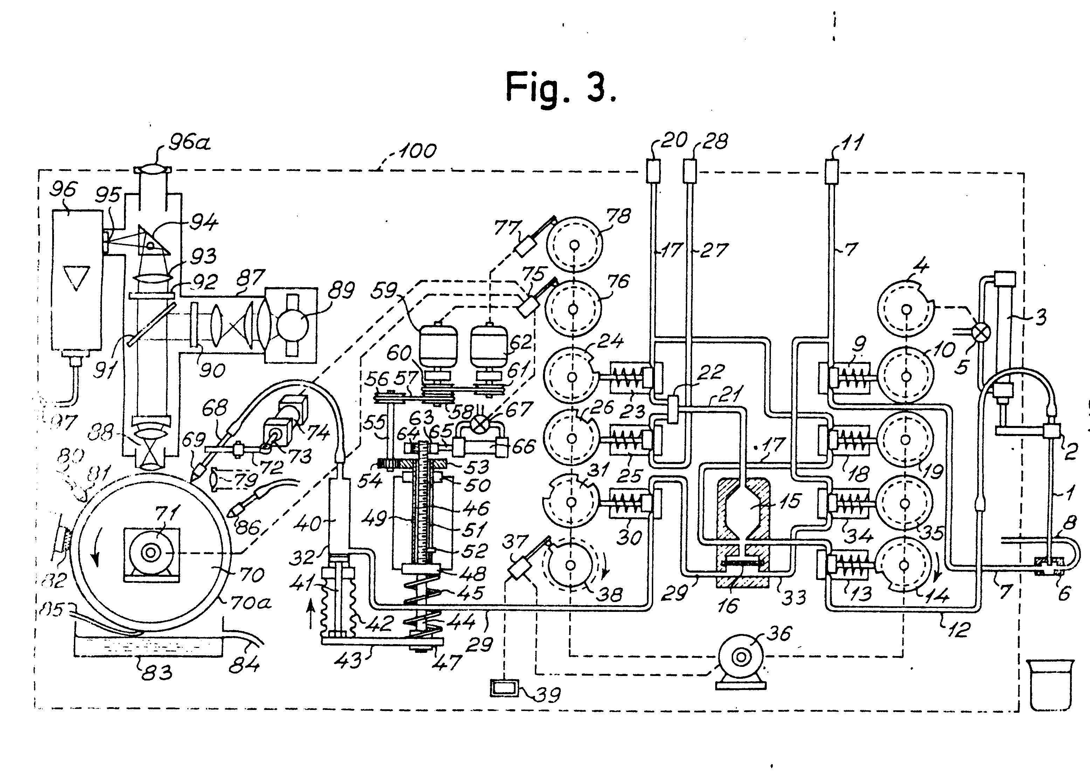

With reference in particular to Fig. 3, counting of bacteria using the apparatus shown is now explained in more detail.

The apparatus shown in the drawings comprises a pipette 1 supported by a support 2, which may be moved up and down by means of a pneumatic cylinder unit 3, the operation of which is controlled by means of a control unit 4, which is suitably a cam actuating a pneumatic valve 5. When moved up and down, the pipette 1 is passed through a jacket 6 communicating with a flushing line 7 and a discharge line 8. Via a valve 9 controlled by a control unit 10, shown as a cam, the flushing line 7 is connected to a water source 11. Through a line 12, which may be closed by means of a valve 13 controlled by a control unit 14, shown as a cam, the pipette 1 is connected with a reservoir 15 arranged above a filter 16, suitably a stainless steel net filter having a mesh size of 10 - 20 μ. The lower part of the reservoir 15 is also in connection with a suction line 17 which is connected to a vacuum source 20, via a valve 18 that is controlled by means of a control unit 19, shown as a cam. The top of the reservoir 15 is connected with a line 21 which, through a branching 22, may be connected partly with the suction line 17 via a valve 23 controlled by a control unit 24 shown as a cam, and partly with a compressed air line 27 via a valve 25 which is controlled by a control unit 26 shown as a cam, the compressed air line 27 communicating with a compressed air source 28. The space below the filter 16 communicates with a connecting line 29 which, via a valve 30 which is controlled by a control unit 31, shown as a cam, communicates with a measuring syringe 32. The space below the filter 16 also communicates with a flushing line 33 which communicates with the flushing line 7 via a valve 34 controlled by a control unit 35 shown as a cam. The control members 4, 10, 14, 24, 26, 31, and 35 operate synchronously in such a way that the desired functions occur at the desired times, such as explained in greater detail below. In Fig. 3, this is, in accordance with a practical embodiment, shown by the fact that the cams 4, 10, 14, 19, and 35 are all arranged on a common shaft, whereas the cams 24, 26, and 31 are arranged on another common shaft, both shafts being rotated synchronously by an electric motor 36 which may be switched on and off by means of a switch 37 which is controlled by a control unit 38, shown as a cam, the operation of which is synchronized with the remaining control members, and which may be actuated by means of a button 39.

The measuring syringe 32 comprises a reservoir 40 which communicates with the connection line 29, and which is emptied by means of a piston 41 which is suitably surrounded by water-filled bellows 42. When the bellows 42 are compressed, the water contained therein is transferred to a reservoir (not shown) from which it returns into the bellow when the bellow is extended. The water in the bellows serves to avoid that any small waste portions leaking from the syringe will form dry deposits and thus disturb the function of the syringe. The piston 41 is mounted on a plate 43 which may be moved up and down by means of a mechanism comprising a drawbar 44 and a pressure spring 45. The drawbar 44 is provided with a thread 46 and is mounted rotatably in a bearing 47 and rotatably and axially displaceably in a bearing 48. The drawbar is surrounded by a hollow shaft 49 mounted in the bearing 48 and in a bearing 50 and provided with a longitudinal groove 51, which receives a pin 52 mounted to the drawbar 44. On the upper part of the shaft 49 is mounted a gear 53 which engages with a gear 54 mounted on a shaft 55 with a pulley 56. The pulley 56 is driven via a V-belt 57 from a pulley 58 on an electric motor 59. On the shaft of the electric motor 59 is also mounted a pulley 60 which, via a V-belt may be driven from a pulley 61 on an electric motor 62 which rotates with a higher speed than the electric motor 59. The pulleys 58 and 60 are combined into a single unit and therefore always move with the same rotational speed. The pulley unit consisting of the pulleys 58 and 60 is mounted on the shaft of the electric motor 59 by means of a pawl mechanism (not shown) and the pulley 61 is mounted on the electric motor 62 by means of a pawl mechanism (not shown). The pawl mechanisms function in such a way that the pulleys 58 and 60 move with the rotational speed of the motor 59 when the motor 62 is inoperative, but move with rotational speed applied from the motor 62 when that electric motor 62 is rotating. The upper part of the drawbar 44 extends through a plate 63 with an elongated opening, one side of which opening is provided with a partial thread 64 and the other side having a smooth wall. The plate 63 is mounted on a piston rod 65 of a pneumatic cylinder 66 controlled by a valve 67.

As mentioned above, the system comprised by the two motors may be replaced with one step motor. Also, the control system comprising the cam-driven valves shown in the drawings is preferably substituted with electromagnetic valves controlled by an electric timer.

The reservoir 40 of the measuring syringe communicates with a tube 68 having a nozzle 69 which, in the position shown, is directed towards a rotating disc 70 which is suitably made of hard glass such as boron-silicate glass, or hard metal such as chromium-coated stainless steel, and which is driven by a motor 71. Suitably, the disc 70 has a diameter of 8 - 12 cm, preferably about 100 mm, and a thickness of about 6 mm and shows a "beveled rim portion (as shown) to form a path 70a of a width of 2 mm on the disc edge. The angle between the longitudinal axis of the nozzle 69 and the tangent to the cylindrical disc edge in the application point is suitably about 60°. The tube 68 is mounted in at one end of a supporting rod 72, the other end of which being pivotally connected to the piston rod 73 of a pneumatic cylinder 74. When the piston rod 73 is moved to its extended position, the nozzle 69 is moved away from the disc edge 70a. The movement of the pneumatic piston is controlled (in a manner not shown) by means of a pneumatic valve which is actuated by means of a switch 75, the switch being controlled by a control unit 76, shown as a cam, and the switch 75 furthermore controlling the pneumatic valve 67 and switching the electric motors 59 and 71 on and off. The two pneumatic cylinders 74 and 76 may be replaced with a single cylinder connected with both the support 72 and the plate 63 if desired. The electric motor 62 is switched on and off by means of a switch 77 which is controlled by a control unit shown as a cam. The cams 76 and 78 are operated synchronously with the remaining cams as indicated in Fig. 3. The electric motors 59 and 71 are driven synchronously to ensure that the suspension is applied in a uniform layer on the path 70a.

For visual assessment of the arrangement of the nozzle 69 in relation to the disc rim 70a, a magnifying glass 79 may be provided, the magnifying glass 79 suitably being adapted to project a picture of the arrangement of the nozzle in relation to the disc edge to a position in the apparatus where the operator can easily study the picture. Through a tube 80 having a nozzle 81, flushing water is continously supplied when the apparatus is working, the flushing water suitably being kept at a temperature of 40 - 50°C and thereby maintaining the disc at this temperature. Below the tube 80 is arranged a sponge 82 which is rotated when the apparatus is operating, and which is in contact with the rim of the disc 70. Below the disc 70 is provided a collecting container 83 with a discharge 84 for sample flushed off and for flushing water, and a suction line 85 for suctioning away a last liquid drop attaching to the disc edge 70a when the disc has been brought to a standstill is placed just below the center of the disc 70. During operation of the apparatus, air is continously supplied through a nozzle 86 for drying the disc prior to application of the suspension sample.

Above the disc 70 a microscope 87 is arranged, the lens 88 of which is arranged exactly in the distance corresponding to exact focusing of the suspension on the disc edge 70a. The lens may suitably be capable of enlarging about 30 - 50 times and has the highest possible aperture, for example 0.8. From a light source 89, suitably a xenon arc lamp, the light is passed through a suitable convex lens system to an excitation filter 90 which allows light up to a certain wavelength to pass. The limitation wavelength of the excitation filter 90 depends on the fluorochrome dye used. When acridine orange is used as fluorochrome dye, the excitation filter should allow light having a wavelength of up to about 550 nm to pass, and when ethidium bromide is used as the fluorochrome dye, the excitation filter should allow the passage of light having a wavelenght of up to about 585 nm. From the excitation filter 5, the excitation light passes through a dichroitic mirror 91, from which it is reflected through the lens 88 and is collected on the suspension film on the disc edge 70a. The light emitted from the fluorescence-stained bacteria passes through the lens 88 and through a dicroitic mirror 91 to an emission filter 92 which, as sharply as possible, delimits the wavelength range in which to be measured. The selection of the wavelength range to which the limitation is to be performed, depends on the fluorochrome dye. When the fluorochrome dye is acridine orange or ethidium bromide, the wavelenght of the emitted light measured is suitably limited to about 595 - 700 nm. From the emission filter, the light passes through a field lens 93 to a pivotal prism 94 from which the light may be passed partly for focusing in a slit 95 (as shown) and partly, subsequent to turning of the prism, to a lens 96a for visual observation of the disc edge through the microscope.

The slit 95 is arranged in such a way and has such a width that the part of the disc edge exposed through the slit corresponds to a slit having a width of 2 - 4μ perpendicular to the disc edge, this slit representing the narrow stationary zone which extends transversely to the direction of rotation of the disc edge. The true width of the slit 95 is 2 - 4μ multiplied with the total enlargement through the microscope. In one practical embodiment in which the transducer for converting the light signals into electrical signals is a photomultiplier, the microscope has a total magnification of 22 times, and the slit has a width of about 60μ, which corresponds to a slit width, referred to the disc edge of about 3μ. When the transducer is a diode array, it is suitable to use a smaller slit width, such as corresponding to about 10 times total magification in the microscope.

Through the slit 95 the picture of the suspension film is exposed to a transducer such as a photomultiplier or diode or diode array 96 in which the light impulses are converted into electrical signals. The photomultiplier may, for example be of the type RCA 4526. The use of a diode as the transducer is preferred, and it is particularly preferred to use a diode array, for example consisting of 4 diodes, each of which serves one fourth of the slit 95. The division of the transducing between several diodes has the advantage that the ratio of signal to noise is increased, considering that the background area exposed to each diode is reduced. The transducer signal may be passed on for further processing from a line exit 97.

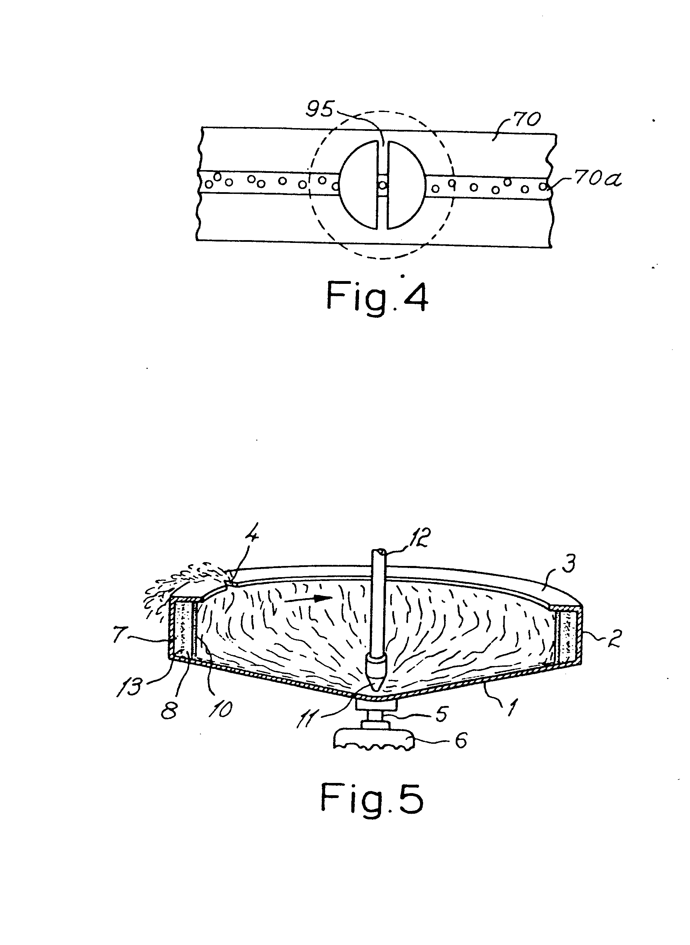

Fig. 4 shows the disc 70 with a disc edge 70a and the suspension of bacteria thereon. Under the conditions selected with respect to the thickness of the suspension film and the dimension and rotational speed of the disc, the bacteria are carried along with the disc virtually as if they were adhered to the disc. Through the slit 95, a single bacterium is noted.

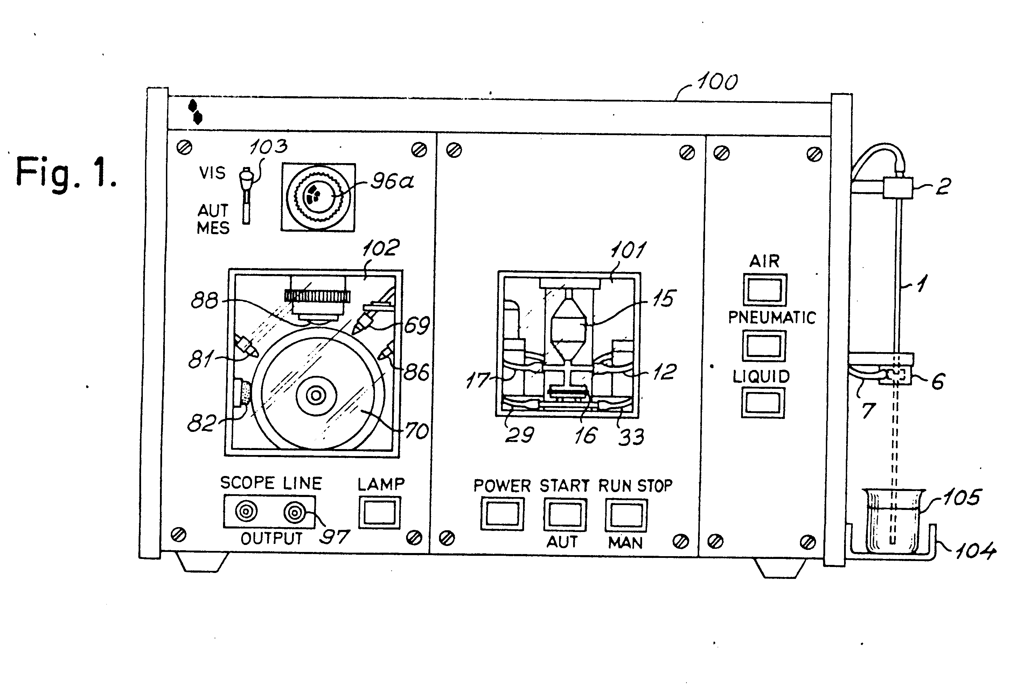

Fig. 1 shows the appearance of a practical embodiment of the apparatus 100 of the invention. Through an inspection window 101, the reservoir 15, the filter 16 and the lines 12, 17, 29, and 33 can be seen so that the function of the sample transfer system can easily be monitored. Through another inspection window 102, the disc 70 and its surroundings can be seen, so that the correct mechanical course of the counting proper can be followed. A shifting lever 103 actuates the prism 94 in the microscope to shift between visual observation through the ocular 96a and projection of the picture on the photomultiplier or diode or diode array 96 through the slit 95. A support 104 carries a beaker 105 with a suspension containing fluorescence-stained bacteria, and the immersed position of the pipette 1 is indicated with dotted lines. The support 104 may be mounted on the apparatus 100, or it may for example be designed as an advancing trough on a sample preparation module which can be placed adjacent to the apparatus 100.

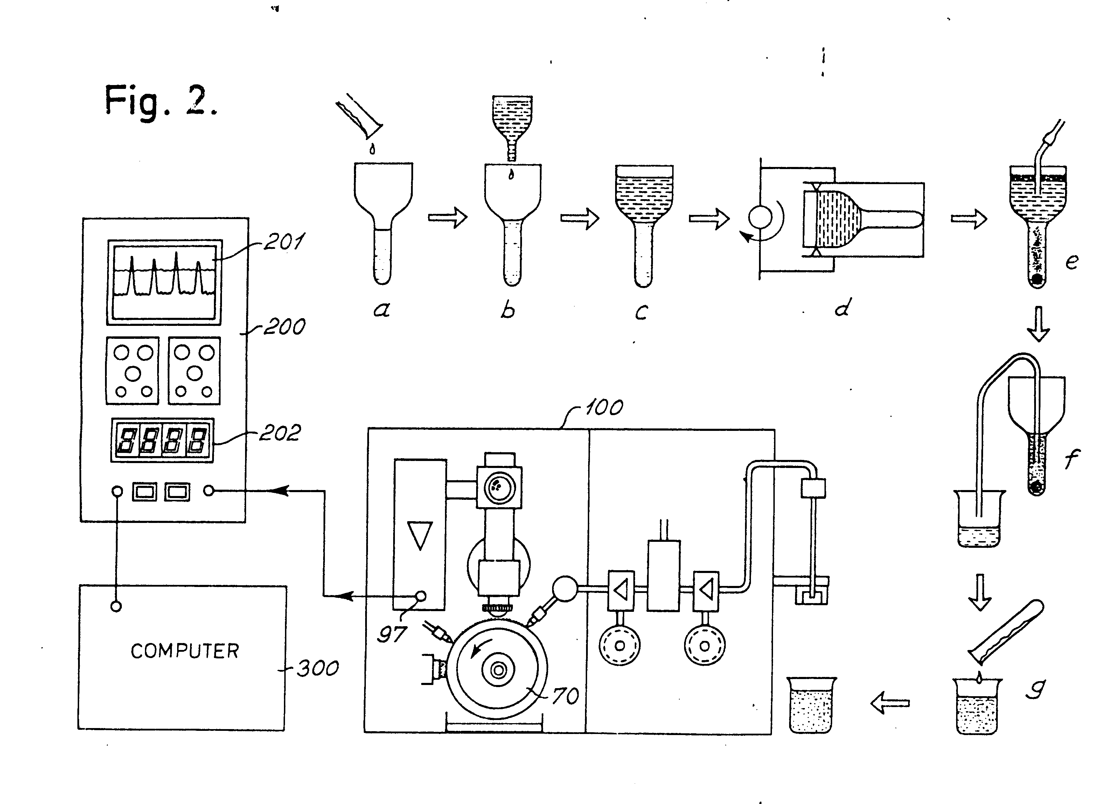

In Fig. 2 situations a - g illustrate the preparation and staining of a sample for counting in the apparatus 100, including establishing the high density liquid gradient column in a centrifuge tube (a), applying sample on top of the liquid column (b and c), centrifuging the system (d), removing sample (e), selectively removing bacteria-containing portion (f), and staining and optionally modifying (g), and 200 is an electronic unit comprising a pulse shaper and discriminator. In the pulse shaper of

26 0008826

the unit 200, the electrical signal withdrawn from the on-line exit 97 in the apparatus 100 is amplified and modified (the modification optionally comprising integration) whereafter the modified and amplified signal is discriminated according to signal size, and signals above a preselected size are counted. From the unit 200, which may also have a digital readout of counted signals as shown by 202,the signal may be passed to a digital computer 300, suitably a microprocessor, which can analyze the signal and separate noise signals, and perform the calculation into number of bacteria per ml of sample. The shape of the modified signals from the pulse shaper and the discriminator level can be visually assessed on an oscilloscope 201. By adjustment by means of suspensions having a known content of bacteria, the discriminator level can be adapted to maximum conformity with for example the total number of bacteria or the content of bacteria determined through the standard method ("viable plate count"). It is also possible to work with several discriminators for better separation between noise and signal, and the use of several discriminators can also serve to distinguish between viable bacteria showing a stronger staining and dead bacteria showing a less intense staining. In the unit 200, the signal from the photomultiplier or the diode og diode array is first passed to an active bandpass filter which only permits passage of signals with the frequencies which may occur when bacteria appear in the slit 95 with the velocity with which they are advanced on the disc edge 70a. A filter, the lower limitation of which is at 5 kHz and the upper limitation of which is at 10 kHz, has been found to be suitable in practice when the slit width, referred to the disc edge, is 3µm, and when the disc has a diameter of 100 mm and rotates with a rotational speed of 15 rpm.

The centrifuge shown in Fig. 5 comprises a centrifuge container 1 having a vertical edge portion 2 and a radially inwardly extending rim portion 3 which optionally has a notch 4. In the embodiment shown, the bottom of the notch 4 determines the minimum radial width of the rim portion. The centrifuge container 1 is mounted on a shaft 5 driven by a motor 6. In the rotating centrifuge container 1, a liquid layer comprising two liquid components 7 and 8 having different densities has a radial thickness substantially equal to the minimum radial thickness of the rim portion. For example, the higher density liquid may be a 30% solution of dextran having a molecular weight of 200,000, and the lower density liquid may be a 6% sucrose solution. A liquid sample 10 is supplied to the center of the centrifuge container through an outlet portion 11 of a supply tube 12. The sample 10 becomes accelerated in the centrifuge container and moves as a thin layer over the liquid layer and is discharged from the centrifuge container at the notch 4. At the interface 13 between the higher density and the lower density liquid components, the baceteria from the sample collect.

Again with reference to Fig. 3, counting of bacteria using the apparatus shown in Fig. 3 will now be described in greater detail:

When the procedure is started, the pipette 1 is immersed in a sample containing fluorescence-stained bacteria, in Fig. 3 designated A. The valve 13 opens simultaneously with the valve 23, and through the vacuum established from the top of the reservoir 15, about 6 ml of sample are aspired into the reservoir. Thereafter, the pipette is lifted by means of the pneumatic cylinder 3, and at the same time, the exterior of the pipette is flushed in that the valve 9 opens so that water passes through the flushing line 7 and the jacket 6 and passes out through the exit line 8. Thereafter, the valves 13 and 23 close, and the valves 30 and 25 open, whereby about 2 ml of liquid are pressed through the filter and passed to the syringe reservoir 40 via the connecting line 29, and a corresponding volume of liquid leaves the nozzle 69 which, at this time, is not pointing towards the disc 70, but is displaced in relation thereto, so that the liquid sprayed out goes into the container 83. When the syringe is filled up with the new sample, the valve 30 closes. At this time there is still elevated pressure in the reservoir 15, and the valve 18 opens so that surplus of liquid in the reservoir is removed via the line 17.

When the syringe reservoir 40 is filled up with the new sample, the switches 75 and 77 actuate the pneumatic cylinders 66 and 74 and start the fast-rotating electric motor 62. Through the actuation of the pneumatic cylinder 74, the nozzle 69 is moved to a position above the disc, and through actuation of the pneumatic cylinder 66, the partial thread 64 in the plate 63 is brought to engage with the thread 46 on the drawbar 44, and the drawbar, and thereby the piston in the measuring syringe, move upwardly. As the fast-rotating electric motor 62 is simultaneously switched on, the first upward movement takes place with the high velocity, whereby liquid contact through the orifice 69 and the disc rim 70a is quickly established. Thereafter, the fast-rotating electric motor 62 is switched off, and the emptying of the measuring syringe is subsequently controlled through the rotational velocity of the electric motor 59 which is synchronized with the rotational velocity of the motor 71. When the piston of the measuring syringe has reached its upper position, the pneumatic cylinder 66 is again actuated so that the partial thread 64 is disengaged from the thread 46, and through the pressure spring 45, the plate 43 and, thereby, the piston 41, are quickly pressed down. Simultaneously with this, the nozzle 69 is removed from the disc edge 70a. At the end of the cyclus of the apparatus, flushing water is introduced from the line 33, goes up through the filter and is aspirated out through the line 21.

Shortly afterwards, the valves 13 and 30 open, whereby flushing water passes out through both measuring syringe and pipette. Immediately at the end of the cyclus, ·the valve 34 closes, but the aspiration from the vacuum source 20 is retained so that the water is emptied back from the syringe and from the pipette, passes up through the filter and is thereafter aspirated out so that the end of the cyclus is an aspiration from the vacuum source. The counting begins as soon as the piston in the measuring syringe has passed the side opening.

For example, a complete cyclus takes 3/4 of a minute, and under the above-stated conditions, the counting time may typically be about 20 seconds. Under these conditions, the size of the sample counted may preferably be about 5 microliter.

For increasing the capacity of the apparatus in an economic way, the flushing and sample transfer system, including the measuring syringe, may be doubled, in other words, two measuring syringes can serve the same disc, the same microscope and the same electronics.

Example 1.

Counting of bacteria in milk.

The centrifugation tube (compare Fig. 2) is made ready by adding, from the bottom and upwardly, equal amounts of:

1) 15 per cent dextran 500 in PBS,

2) 12 per cent dextran 500 in PBS,

3)  7 per cent dextran 500 in PBS, and

4) 4,5 per cent sodium diatrizoate in PBS of half concentration,

so that the lower column-part of the chamber is filled up with the gradient material.

2 ml of raw milk (incubated for 20 hours at room temperature) is passed through a cannula (0.5 x 16 mm) during 5 seconds to 12 ml PBS containing 1 per cent EDTA (temperature 50°C), and the resulting mixture is placed in the conical part of the centrifugation tube on top of the gradient material. Thereafter, the sample is centrifuged for 4 minutes and 20 seconds, whereafter the content of the conical part is discarded and the upper 95 per cent of the gradient material are transferred to 4 ml N-broth containing $25 \times 10^{-4}$ Anson·units of alkalase, 750 international units of penicillin, and 10 per cent sucrose, and mixing is performed with a Pasteur-pipette. The mixture is allowed to stand for 30 minutes at 30°C, whereafter it is transferred via a cannula (0.5 x 16 mm) during 8 seconds to 9 ml 0.2 molar borate buffer containing 1.4 per cent formaldehyde, 0.01 per cent Triton X-100, 40 microliter 0.1 per cent acridine orange and 0.4 per cent EDTA. The buffer pH-adjusted in such a way that the pH of the final mixture  is 9.2. This mixture is allowed to stand for 20 seconds, whereafter it is counted using the counting module illustrated in the drawings.

In the manner described above two different samples, A and B, are prepared.

A shows a count of 324 impulses, which through an algorithm is transformed to a count of $3.9 \times 10^5$ bacteria per mm (viable plate count on the same sample shows $3.8 \times 10^5$ bacteria per ml).

Sample B shows a count of 851 impulses corresponding to 2.6 x $10^6$ bacteria per ml (viable plate count on the same sample shows 3.0 x $10^6$ per ml).

Example 2.

Counting of bacteria in urine.

From a urine sample (midstream urine) which has been kept for less than 12 hours at 4°C subsequent to withdrawal, a portion of 6 ml is taken. To these 6 ml is added 1 ml of 1 per cent papain in hexamethaphosphate buffer having a pH of about 7 (4 per cent sodium hexamethaphosphate and 12 per cent urea), and the mixture is allowed to stand for 7 minutes at 40°C. After incubation, 5 ml of borate buffer having a pH of 9.2 and 36 microliter of 0.1 per cent acridine orange solution are added, and after 20 seconds the sample is counted with the apparatus described in connection with Fig. 3.

In the above manner two samples, A and B, are prepared with urine which has been store for less than 12 hours at 4°C.

A shows a count of 110 impulses corresponding to 4.4 x $10^4$ bacteria per ml (viable plate count on the same sample shows 4.5 x $10^4$ bacteria per ml).

Sample B shows a count of 285 impulses corresponding to 1.1 x $10^5$ bacteria per ml (viable plate count on the same sample shows 1.5 x $10^5$ bacteria per ml).

Example 3.

Counting of bacteria in milk.

The centrifugation tube (compare Fig. 2) is made ready by adding, from the bottom and upwardly, equal amounts of: 1) 30 per cent dextran T70 in 0.01M Tris buffer with 0.2 per cent NaCl,
2) 6 per cent dextran T70 in 0.01M Tris buffer with 0.2 per cent NaCl,

so that the lower column-shaped part of the chamber is filled up with the gradient material.

4 ml of raw milk is admixed with a mixture consisting of 12 ml of demineralized water and 1 ml of a solution of digitonin (0.1 per cent in 50% ethanol). The temperature of the mixture is 50°C. The milk solution is homogenized through passage through a cannula (0.5 x 12 mm) at a rate of about 2 ml/second. Thereafter, 1.2 ml of a tenfold concentrated PBS with 3 per cent EDTA is added, and the resulting mixture is placed in the conical part of the centrifugation tube on top of the gradient material. The sample is centrifuged for 7 minutes, whereafter the contents of the conical part of the centrifugation tube plus the uppermost third of the gradient material are discarded, while the rest of the gradient material (0.5 ml) is transferred to 2.5 ml 0.1 per cent peptone with 0.2 per cent NaCl and 0.01 Anson unit/ml of Alcalase (NOVO) (subtilisin) (final pH about 9), and mixing is performed by homogenization with Pasteur pipette. The mixture is allowed to stand for 50°C for 10 minutes, whereafter 2.5 ml 0.1M borate buffer containing 1.4 per cent formaldehyde and 15 µliter 0.1 per cent acridine orange are added. The buffer is pH-adjusted in such a way that the pH of the final mixture is 9.2. This mixture is allowed to stand for 20 seconds whereafter it is counted using the counting module illustrated in the drawings.

In this manner, 28 milk samples were treated and counted, and the results of the countings were correlated to plate count (plate count agar from Oxoid; incubation for 3 days at 30°C, surface inoculation by means of "spiral plate maker").

The correlation coefficient was calculated to 0.945, the slope of the regression line was 0.645, the intersection with the y-axis was 1.509, the deviation in the x-axis was 0.28, and the deviation in the y-axis was 0.39.

Claims.

1. A method for counting bacteria in a bacteria-containing suspension, said method comprising staining said bacteria with a fluorochrome dye, rotating a circular-cylindrical surface part about its axis, applying a film of suspension containing the stained bacteria on the said rotating surface part, exposing said film to fluorescence-activating radiation, detecting light signals emitted from said stained bacteria, transforming said light signals to electrical signals, selecting said electrical signals exceeding a predetermined size, and counting said selected signals.

2. A method as claimed in claim 1 wherein the film of suspension applied on the rotating surface part has an average thickness of 2 - 7 µm, in particular 4 - 6 µm, during the counting of the selected signals.

3. A method as claimed in claim 2 in which the width of the film is about 400 - 800 µm.

4. A method as claimed in claim 1 in which the variation of the radius of the rotating surface part along its circumference is at the most 2 µm, preferably at the most 1 µm.

5. A method as claimed in claim 1 in which the rotating surface part has a diameter of 8 - 12 cm and rotates at about 10 - 60, preferably 10 - 40, rpm.

6. A method as claimed in claim 5 in which the rotating surface part has a diameter of about 100 mm and rotates at about 15 rpm.

7. A method as claimed in claim 1 in which the zone of the suspension film from which light signals are detected is a stationary zone extending transversely to the direction of rotation of the surface part and has a width of about 2 - 4 μm, preferably 3 μm.

8. A method as claimed in claim 1 in which the staining of the bacteria is performed with acridine orange or ethidium bromide.

9. A method for fluorescence staining of bacteria in suspension, preferably in connection with the method claimed in claim 1, comprising performing a modification treatment of the suspension to increase the intensity of the fluorescence staining of the bacteria in comparison with the background and/or to reduce the number of stainable particles which are not bacteria.

10. A method as claimed in claim 9, comprising modifying the suspension by incubation with a proteolytic enzyme prior to the staining.

11. A method as claimed in claim 9, comprising incubating the bacteria, prior to the staining, in a nutrient medium containing such antibiotic under such conditions that the bacteria to be stained will grow, but will not divide to any substantial extent.

12. A method as claimed in claim 9 in which the staining is performed with acridine orange, comprising adding formaldehyde to the suspension simultaneously or substantially simultaneously with the acridine orange.

13. A method for separating bacteria from a bacteria-containing liquid sample, the bacteria concentration of which is to be determined, preferably for preparing a suspension to be subjected to the counting operation claimed in claim 1, said method comprising applying the sample on a layer of a liquid having a higher density than the sample, centrifuging the system constituted by the said layer and the sample applied thereon so as to move bacteria from the sample into a portion of the liquid layer and thereafter separating the liquid layer or the bacteria-containing portion thereof.

14. A method as claimed in claim 13 in which the liquid layer comprises a discontinuous or continuous density gradient.

15. A method as claimed in claim 14 in which the gradient comprises a phase of lower viscosity and a phase of higher viscosity, the phase of lower viscosity being adjacent to the sample.

16. A method as claimed in claim 13 in which said liquid layer comprises at least two liquid components having different densities, said bacteria being moved to a portion of said liquid layer at the interface between said liquid components.

17. A method as claimed in claim 16 in which said system is centrifuged in a dish-like centrifuge container having an upper opening defined by a radially inwardly extending rim portion having a minium radial width substantially equal to the radial thickness of said liquid layer, said sample being supplied to the bottom of said centrifuge container and the centrifuged sample being discharged through said upper opening.

18. A method as claimed in claim 17 wherein said sample is supplied to the centrifuge container through a supply tube extending through said upper opening of the centrifuge container to a position adjacent to the bottom thereof, preferably at a substantially uniform rate.

19. An apparatus for counting fluorescence-stained bacteria in a suspension, comprising

a rotatable suspension-receiving member having a circular-cylindrical surface part,

suspension application means for applying a film of said suspension on said cylindrical surface part, radiation means for irradiating said film to activate fluorescence from the stained bacteria,

transducer means for transforming light signals into electrical signals,

counting means for counting electrical signals,

means for passing light signals emitted by the fluorescence-stained bacteria to said transducer means, and discriminator means for discriminating the electrical signals produced by said transducer means according to size so as to pass only signals exceeding a predetermined value to the counting means.

Fig. 1.

# Fig. 2.

COMPUTER

*201*

*200*

*202*

*300*

*100*

*97*

*70*

*a*  *b*  *c*  *d*  *e*

*f*  *214*

*g*

0008826

# Fig. 3.

0008826

0008826

Fig.4

Fig.5